# EUROPEAN PATENT APPLICATION

(11) **EP 4 793 341 A1**
(43) Date of publication of application: **19.08.2026**
(21) Application number: 23955176.5
(22) Date of filing: 13.10.2023
(51) Int. Cl.: C12G 3/02, C12M 1/16, C12M 1/04, C12M 1/00

(54) **DEVICE FOR PRODUCING STARTER AND METHOD FOR PRODUCING STARTER**

(71) Applicant: Fujiwara Techno-Art Co., Ltd., Okayama 701-1133 (JP); Fujiwara Techno-Art (Shanghai) Co., Ltd., Shanghai 200336 (CN)
(72) Inventor: KARIYAMA, Masahiro, Okayama-shi, Okayama 701--1133 (JP); MORI, Akira, Okayama-shi, Okayama 701--1133 (JP); YAMAMOTO, Tatsunori, Okayama-shi, Okayama 701--1133 (JP); WANG, Gang, Jiangmen, Guangdong 529156 (CN); YANG, Yupeng, Jiangmen, Guangdong 529156 (CN); CHAN, Siu Keung, Jiangmen, Guangdong 529156 (CN)
(74) Representative: Herrero & Asociados, S.L.
(86) International application number: PCT/CN2023/124400
(87) International publication number: WO 2025/076796

(57) **Abstract**

A production apparatus (1) for a seed mold includes a steaming and cooling device (2) that steams and cools a raw material (60) in batches, a ventilated solid cultivation device (50) that cultures the raw material (60) by accumulating the raw material (60) on a culture bed (52) capable of being ventilated, a heaping device (30) that conveys the raw material (60) discharged from the steaming and cooling device (2) and supplies the raw material (60) to the ventilated solid cultivation device (50), a water adding device (55) for culture that supplies water to the raw material (60) on the culture bed (52), and a culture sterile air supply device (70) that ventilates sterile air to the raw material (60) on the culture bed (52), and the sterile air passes through the raw material on the culture bed (52).

## Description

### TECHNICAL FIELD

The present disclosure relates to a production apparatus for a seed mold and a production method for a seed mold in which a raw material is processed to produce a seed mold. The seed mold is spores of koji mold or a solid cultivation containing spores of koji mold, and is used as a starter for producing koji, which is essential for production of fermented foods. The seed mold is also used for production of enzymes or for adding functionality to food or feed.

### BACKGROUND

In production of a seed mold, it is important to produce the seed mold that is not contaminated by Misc. Bacteria (Miscellaneous Bacteria) through culture in an aseptic condition. Patent Literature 1 discloses a production apparatus for a seed mold. The term "inoculum" used in Patent Literature 1 is the same as the term "original seed mold" in this specification. The production apparatus for a seed mold described in Patent Literature 1 is formed by a pressure vessel having a horizontal cylindrical shape, a original seed mold supply device arranged in communication with the pressure vessel, and a pressure device in communication with the original seed mold supply device. In the disclosed production apparatus for a seed mold, the pressure device is activated to supply the original seed mold to the pressure vessel while keeping the interior of the pressure vessel at a pressure higher than atmospheric pressure.

In the pressure vessel having a horizontal cylindrical shape described above, a seed mold culture medium shelf is mounted on rails, and a tray on which a culture medium consisting of bran or other grain is put is mounted on each board of the seed mold culture shelf. The production apparatus for a seed mold described in Patent Literature 1 distributes the original seed mold onto the culture medium on the tray under pressure and therefore has an advantage that it is difficult for Misc. bacteria to enter the apparatus from outside and the original seed mold can be exclusively cultured.

### Citation List

### Patent Literature

Patent Literature 1: Japanese Patent Application Laid-open No. 2000-197475

### SUMMARY

As described above, in production of a seed mold, it is important to culture the seed mold in an aseptic condition. However, it is also important to perform uniform inoculating, and appropriate temperature control and water content control during culture. In general, as with the production apparatus for a seed mold described in Patent Literature 1, a horizontal thin layer static culture is performed in which a solid cultivation raw material adjusted to have a predetermined water content is heaped onto a tray in an airtight vessel in a thin layer, and steaming, cooling, inoculating and culture are performed in the airtight vessel.

However, since the raw material on the tray is in the shape of a thin layer, the apparatus needs to be upsized to mass-produce the seed mold, and there is a problem with workability because of the manual operations, such as heaping and koji removal. In addition, since a plurality of trays are mounted on each shelf board, and the raw material is accumulated on each tray, uniform inoculating is difficult. To be specific, there is a problem that the number of spores of the produced seed mold significantly varies in the depth direction of the accumulation because the original seed mold is likely to come into contact with an upper part of the raw material on each tray and is less likely to come into contact with a lower part of the raw material. Furthermore, splinkling is needed during culture in order to produce a seed mold containing a large number of spores, and there is a problem that the water content of the raw material varies in the depth direction of the accumulation for the same reason as for the problem with the contact of the original seed mold described above.

To solve the problems of the prior art described above, an object of the present disclosure is to provide an apparatus and a method for efficiently producing a seed mold that are suitable for automation of the apparatus and mass production of a seed mold and can reduce variations of the number of spores of the produced seed mold in the depth direction of the accumulated raw material.

To attain the object described above, a production apparatus for a seed mold according to the present disclosure includes a steaming and cooling device that steams and cools a raw material in batches, a ventilated solid cultivation device that cultures the raw material by accumulating the raw material on a culture bed capable of being ventilated, a heaping device that conveys the raw material discharged from the steaming and cooling device and supplies the raw material to the ventilated solid cultivation device, a water adding device for culture that supplies water to the raw material on the culture bed, and a culture sterile air supply device that ventilates sterile air to the raw material on the culture bed, and the sterile air passes through the raw material on the culture bed.

A production method for a seed mold according to the present disclosure includes a steaming and cooling step of steaming and cooling a raw material in batches using a steaming and cooling device, a culture step of accumulating and culturing the raw material on a culture bed capable of being ventilated using a ventilated solid cultivation device, and a heaping step of conveying the raw material subjected to the steaming and cooling step and supplying the raw material to the ventilated solid cultivation device using a heaping device, the heaping step being between the steaming and cooling step and the culture step, water is supplied to the raw material on the culture bed in the culture step, and sterile air is passed through the raw material on the culture bed.

With the production apparatus for a seed mold and the production method for a seed mold according to the present disclosure described above, various effects can be achieved as described below, and a seed mold can be efficiently produced. According to the present disclosure, a production apparatus is used which generally includes a steaming and cooling device, a heaping device and a ventilated solid cultivation device. The batch steaming and cooling of the raw material by the steaming and cooling device can be automated, and the heaping of the raw material by the heaping device can also be automated. In the ventilated solid cultivation device, the ventilation of the raw material on the culture bed can be automated, and the koji removal can also be automated with a discharging screw. That is, each device used in the present disclosure can be automated, the heaping and the koji removal can be automated in particular, and the present disclosure is suitable for automation.

The culture of the raw material is performed by using the ventilated solid cultivation device and accumulating the raw material on the culture bed. Therefore, the accumulation height of the raw material can be increased, and the present disclosure is suitable for mass production of seed mold. In addition, since sterile air passes through the raw material on the culture bed during the culture, the temperature can be controlled to reduce the variation of the substance temperature with the depth in the accumulation, by controlling the air temperature and air humidity of the sterile air. In addition, since the raw material on the culture bed can be turned, the variation of the number of spores in the produced seed mold in the depth direction of the accumulation can be reduced.

According to the present disclosure, since water is supplied to the raw material on the culture bed, the water content of the raw material can be appropriately adjusted, and a seed mold containing a large number of spores can be produced. In addition, since the heaping device and the ventilated solid cultivation device are separate devices, the raw material can be uniformly seeded in the heaping device in advance and then be heaped to the ventilated solid cultivation device.

The production apparatus for a seed mold and the production method for a seed mold according to the present disclosure described above are preferably configured as described below. In the production apparatus for a seed mold according to the present disclosure described above, preferably, the steaming and cooling device is of a jacket type including an inner tank and an outer tank surrounding the inner tank, the inner tank being cooled by a coolant supplied between the inner tank and the outer tank. In the production method for a seed mold according to the present disclosure described above, preferably, the steaming and cooling device used in the steaming and cooling step is of a jacket type including an inner tank and an outer tank surrounding the inner tank, the inner tank being cooled by a coolant supplied between the inner tank and the outer tank. Cooling with the steaming and cooling device of the jacket type does not involves forcedly passing air through the raw material, so that there is no risk of contamination by Misc. bacteria.

The production apparatus for a seed mold according to the present disclosure described above preferably further includes a raw material processing sterile air supply device that supplies sterile air to the steaming and cooling device. In the production method for a seed mold according to the present disclosure described above, preferably, sterile air is supplied to the raw material in the steaming and cooling step. With such configurations, the raw material can be cooled in a sterile air atmosphere.

In the production apparatus for a seed mold according to the present disclosure described above, preferably, an interior of the ventilated solid cultivation device is kept at a positive pressure by the sterile air supplied by the culture sterile air supply device. In the production method for a seed mold according to the present disclosure described above, preferably, an interior of the ventilated solid cultivation device is kept at a positive pressure by supplying the sterile air to the interior of the ventilated solid cultivation device. With such configurations, entry of Misc. bacteria can be prevented while performing an substance temperature control for controlling the substance temperature of the raw material to a target substance temperature.

In the production apparatus for a seed mold according to the present disclosure described above, preferably, an interior of the steaming and cooling device is kept at a positive pressure by the sterile air supplied by the raw material processing sterile air supply device. In the production method for a seed mold according to the present disclosure described above, preferably, an interior of the steaming and cooling device is kept at a positive pressure by supplying the sterile air to the interior of the steaming and cooling device. With such configurations, the pressure in the steaming and cooling device can be prevented from being negative, so that the risk of contamination by Misc. bacteria due to sucking of the outside air into the steaming and cooling device can be eliminated.

In the production apparatus for a seed mold according to the present disclosure described above, preferably, the seed mold after culture is dried by the sterile air supplied by the culture sterile air supply device. In the production method for a seed mold according to the present disclosure described above, preferably, the seed mold after culture is dried by supplying the sterile air. The drying can prevent growth of Misc. bacteria and allow long-term storage.

The production apparatus for a seed mold according to the present disclosure described above preferably further includes a turning machine that turns the raw material on the culture bed, and the water adding device for culture preferably supplies water to the raw material while the raw material is turned by the turning machine. In the production method for a seed mold according to the present disclosure described above, preferably, water is supplied to the raw material while turning is performed to turn the raw material on the culture bed. With such configurations, the raw material can be prevented from forming lumps, which make it difficult to control the substance temperature of the raw material to an intended substance temperature, and water content control can be performed to prevent the water content of the raw material from decreasing in the culture step and becoming unsuitable for sporulation.

In the production apparatus for a seed mold and the production method for a seed mold according to the present disclosure described above, preferably, air conditioning of an interior of the ventilated solid cultivation device by supplying the sterile air is one-way. With such configurations, the air having passed through the raw material is not circulated in the ventilated solid cultivation device but is discharged to the outside of the device, so that the culture can be achieved in a more aseptic condition than a circulation system.

The production apparatus for a seed mold according to the present disclosure described above preferably further includes a water adding device for the steaming and cooling device that splinkles water onto the raw material in the steaming and cooling device or a water adding device for the heaping device that splinkles water onto the raw material in the heaping device, and a water content of the raw material in the steaming and cooling device or the raw material in the heaping device and a water content of the raw material on the culture bed are adjustable by adjusting an amount of water from the water adding device for the steaming and cooling device or the water adding device for the heaping device and an amount of water from the water adding device for culture. The production method for a seed mold according to the present disclosure described above preferably further includes a step of supplying water to the raw material in the steaming and cooling step or a step of supplying water to the raw material in the heaping step, and a water content of the raw material in the steaming and cooling step or the raw material in the heaping step and a water content of the raw material in the culture step are adjustable by adjusting an amount of the water supplied in the steaming and cooling step or an amount of the water supplied in the heaping step and an amount of the water supplied in the culture step. With such configurations, a target water content of the raw material immediately after the heaping of the raw material onto the culture bed in the ventilated solid cultivation device can be ensured, and the water content of the raw material in the culture step can be adjusted.

In the production apparatus for a seed mold according to the present disclosure described above, preferably, the water adding device for the steaming and cooling device, the water adding device for the heaping device and the water adding device for culture are capable of adjusting a pH of water to be splinkled. In the production method for a seed mold according to the present disclosure described above, preferably, a pH of the water supplied in the steaming and cooling step, the heaping step and the culture step is adjusted. The pH adjustment allows the seed mold to be produced in a more aseptic condition.

The present disclosure has the effects described above. In summary, each device used in the present disclosure can be automated, and the present disclosure is suitable for automation. Culture of the raw material is performed by accumulating the raw material on the culture bed, so that the present disclosure is suitable for mass production of the seed mold. In addition, since sterile air passes through the raw material on the culture bed during culture, temperature control can be performed to reduce variations of the substance temperature in the depth direction of the accumulation. And since the raw material on the culture bed can be turned, variations of the number of spores of the produced seed mold in the depth direction of the accumulation can be reduced. According to the present disclosure, since water is supplied to the raw material on the culture bed, the water content of the raw material can be appropriately adjusted, and a seed mold containing a large number of spores can be produced. In addition, since the heaping device and the ventilated solid cultivation device are separate devices, the raw material can be uniformly seeded in the heaping device in advance and then be heaped to the ventilated solid cultivation device.

### BRIEF DESCRIPTION OF THE DRAWINGS

Fig. 1 is a diagram illustrating a general configuration of a production apparatus for a seed mold according to an embodiment of the present disclosure;
Fig. 2 is a flowchart illustrating a production process for a seed mold according to the embodiment of the present disclosure;
Fig. 3 is a diagram illustrating a steaming and cooling device into which a raw material is being put according to the embodiment of the present disclosure;
Fig. 4 is a diagram illustrating the steaming and cooling device in a splinkling and mixing step according to the embodiment of the present disclosure;
Fig. 5 is a flowchart specifically illustrating the splinkling and mixing step according to the embodiment of the present disclosure;
Fig. 6 is a diagram illustrating the steaming and cooling device in a steaming step according to the embodiment of the present disclosure;
Fig. 7 is a flowchart specifically illustrating a steaming step according to the embodiment of the present disclosure;
Fig. 8 is a diagram illustrating the steaming and cooling device in a cooling step according to the embodiment of the present disclosure;
Fig. 9 is a flowchart specifically illustrating the cooling step according to the embodiment of the present disclosure;
Fig. 10 is a diagram illustrating the steaming and cooling device when a positive pressure is produced in the whole of the interior of the production apparatus according to the embodiment of the present disclosure;
Fig. 11 is a flowchart specifically illustrating a heaping step according to the embodiment of the present disclosure;
Fig. 12 is a flowchart illustrating an overview of a culture step and a drying step according to the embodiment of the present disclosure;
Fig. 13 is a flowchart specifically illustrating an substance temperature control in the culture step according to the embodiment of the present disclosure; and
Fig. 14 is a flowchart specifically illustrating the drying step subsequent to the culture step according to the embodiment of the present disclosure.

### DETAILED DESCRIPTION OF THE EMBODIMENTS

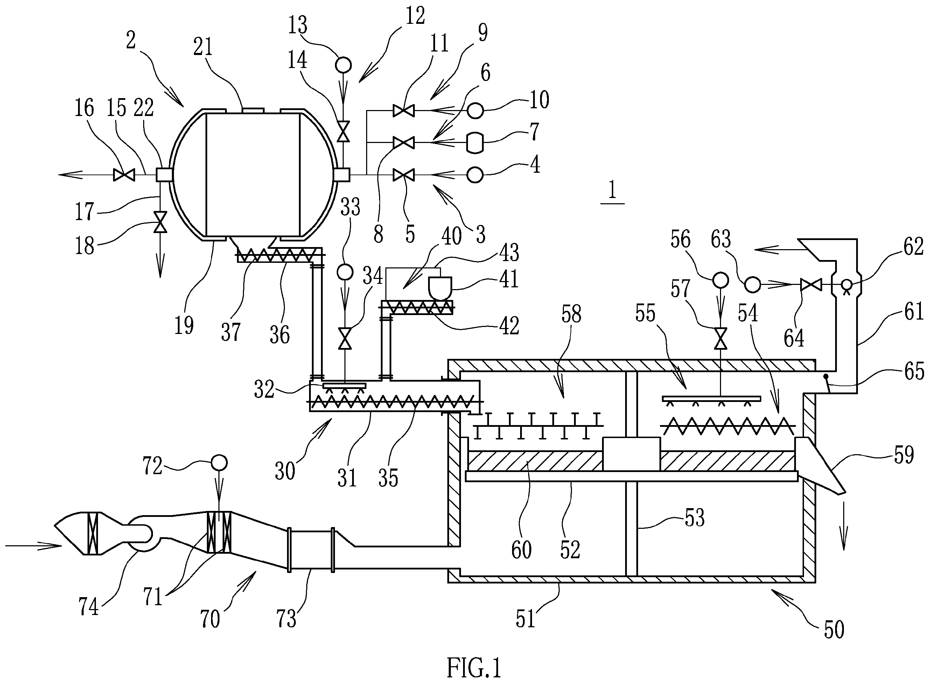

In the following, an embodiment of the present disclosure will be described with reference to the drawings. Fig. 1 is a diagram illustrating a general configuration of a production apparatus 1 for a seed mold (referred to simply as a producing apparatus 1, hereinafter) according to an embodiment of the present disclosure. An overview of the producing apparatus 1 will first be described with reference to Fig. 1. In Fig. 1, the producing apparatus 1 generally includes a steaming and cooling device 2, a heaping device 30, and a ventilated solid cultivation device 50. The steaming and cooling device 2 is provided with a raw material processing sterile air supply device 3, a steam supply device 6, a water adding device 9 for the steaming and cooling device, and a cooling water supply device 12. The heaping device 30 includes a conveyor device 36 and a mixing device 31, and is provided with a water adding device 32 for the heaping device and a inoculating device 40. The ventilated solid cultivation device 50 includes a turning machine 58 and a discharging screw 54, and is provided with a culture sterile air supply device 70 and a water adding device for culture 55.

The steaming and cooling device 2 steams and cools a raw material in batches. That is, in the steaming and cooling device 2 with the raw material put therein, a splinkling and mixing step, a steaming step, and a cooling step proceeds in succession. These steps will be described in detail later. The raw material discharged from the steaming and cooling device 2 is supplied to the heaping device 30. In the heaping device 30, the raw material is mixed with a original seed mold supplied from the inoculating device 40 while the raw material is conveyed. The original seed mold is produced in another apparatus, and the term "original seed mold" is used for discrimination from the term "seed mold" produced in the producing apparatus 1 according to this embodiment.

The raw material conveyed in the heaping device 30 is supplied to the ventilated solid cultivation device 50. Fig. 1 illustrates a raw material 60 accumulated on a culture bed 52, and the culture occurs in this state. The culture bed 52 can be ventilated, and sterile air from the culture sterile air supply device 70 passes through the culture bed 52. In addition, the culture bed 52 can rotate about a central post 53, and water is supplied from the water adding device 55 for culture to the raw material 60 on the culture bed 52.

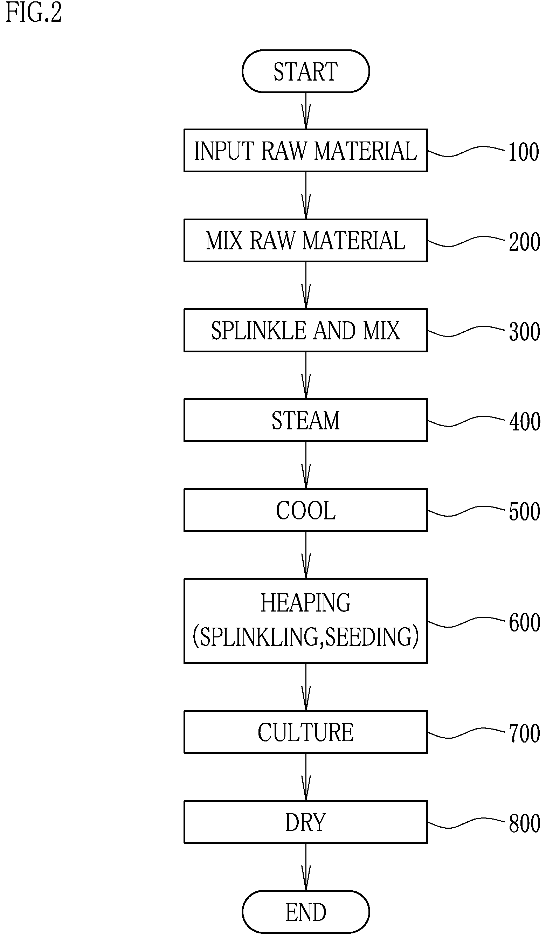

Fig. 2 is a flowchart illustrating a production process for a seed mold using the producing apparatus 1 according to this embodiment. In the following, the production process according to this embodiment will be described step by step. In Fig. 2, steps from a raw material input step (Step 100) to the cooling step (Step 500) occur in the steaming and cooling device 2 illustrated in Fig. 1. In Fig. 1, a main part of the steaming and cooling device 2 is constituted by a rotating drum 19. Fig. 1 illustrates the interior of the drum 19. Although only simply illustrated in Fig. 1, the drum 19 is of the jacket type, which includes an inner tank and an outer tank surrounding the inner tank, and the inner tank is cooled by a coolant supplied between the inner tank and the outer tank. In the cooling of the raw material in the jacket manner, in which air isn't forcedly passed through the raw material for cooling, there is no risk of contamination by Misc. bacteria.

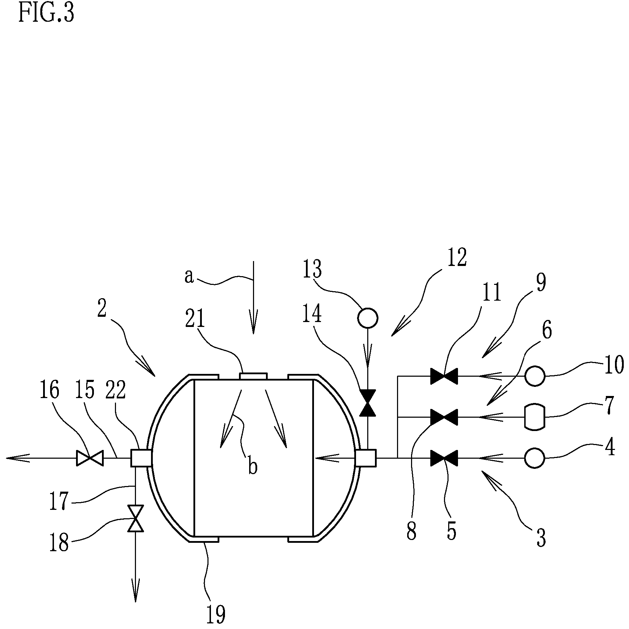

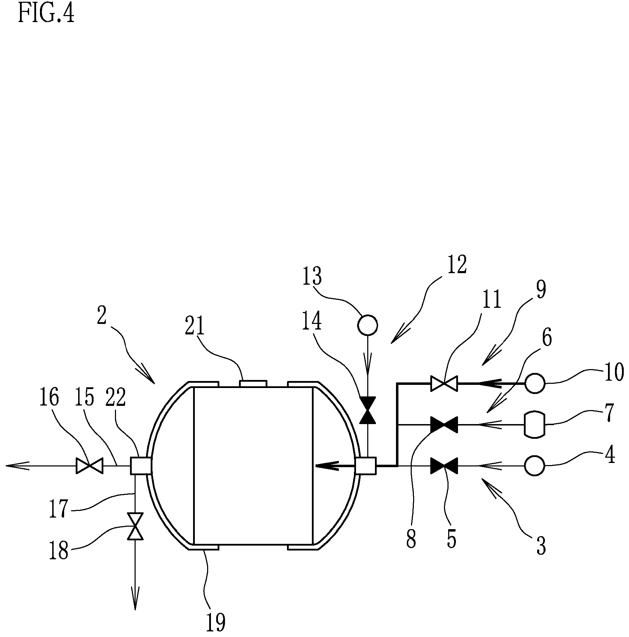

After the start of the production process, first, a raw material (for example, 2000 kg of bran) of the seed mold is put in the drum 19 of the steaming and cooling device 2 in Fig. 1 (Step 100 in Fig. 2). The raw material is not limited to bran, but may be a grain or a processed grain. Furthermore, an additive commonly used for production of the seed mold may be added to the raw material as a main ingredient. Fig. 3 illustrates the steaming and cooling device 2 in Fig. 1 (Figs. 4, 6, 8 and 10 also illustrate the same). In Fig. 3, input of the raw material (indicated by the arrows a and b) is performed by opening a manhole 21 when the manhole 21 is located at the top of the drum 19. The manhole 21 is configured with a lid that opens and closes an opening, and the lid is removed when the raw material is input. After the input of the raw material, the manhole 21 is closed, and the raw material is mixed (Step 200 in Fig. 2). The mixing of the raw material is achieved by rotating the drum 19 about a rotation axis 22.

In the state in Fig. 3, a sterile air regulating valve 5, a steam regulating valve 8, a water regulating valve 11, and a cooling water regulating valve 14 are closed, and these valves are solidly filled. In Figs. 4, 6, 8 and 10, the closed valves are also solidly filled.

Fig. 4 illustrates the steaming and cooling device 2 in the splinkling and mixing step (Step 300 in Fig. 2). In the splinkling and mixing step, water is splinkled on the raw material while rotating the drum 19 to mix the raw material. The splinkling occurs not only in the drum 19 of the steaming and cooling device 2 but also in the heaping device 30 and the ventilated solid cultivation device 50 as described later. The water content of the raw material is appropriately controlled by the splinkling, thereby producing a larger number of spores of the seed mold.

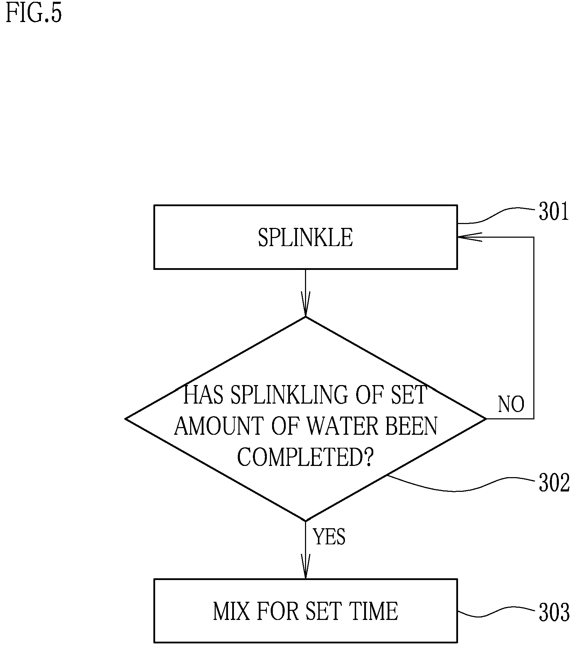

The splinkling is performed by the water adding device 9 for the steaming and cooling device in the state in Fig. 4. Specifically, the splinkling is performed using water supplied from a water supply source 10 to the interior of the drum 19 via the water regulating valve 11. In Fig. 4, the supply path of the water is indicated by a thick line. Fig. 5 is a flowchart specifically illustrating the splinkling and mixing step. After the start of the splinkling, the splinkling is ended when splinkling of a set amount (for example, 2000 L) of water is completed, and then the raw material is mixed for a set time (for example, 20 minutes) (Steps 301 to 303 in Fig. 5).

The amount of water splinkled is controlled with a flowmeter. When steam is supplied into the drum 19 in the subsequent steaming step (Step 400 in Fig. 2), the steam condenses on the inner surface of the drum 19 and is absorbed by the raw material. The amount of the condensate varies with the temperature of the drum before the steaming, so that the amount of the condensate that occurs in the steaming is estimated from the temperature of the drum 19 before the steaming, and the amount of water splinkled is determined by considering the amount of the condensate. A target value of the water content of the raw material after the splinkling needs to be appropriately determined for the following reasons. If the water content of the raw material after the splinkling is too high, the raw material tends to form lumps, which take a longer time to totally cool down in the subsequent cooling step, so that the cooling step takes a longer time. In addition, in the inoculating performed in a heaping step (Step 600 in Fig. 2), an inner part of the lump is not seeded, which is disadvantageous for uniform production of the seed mold. To the contrary, if the water content is too low, the raw material may partially fail to absorb water, and the part of the raw material that has not absorbed water cannot be steamed with reliability. Therefore, the target value of the water content of the raw material after the splinkling preferably falls within a range from 30% to 75%, and preferably falls within a range from 50% to 60% when the main ingredient of the raw material is bran.

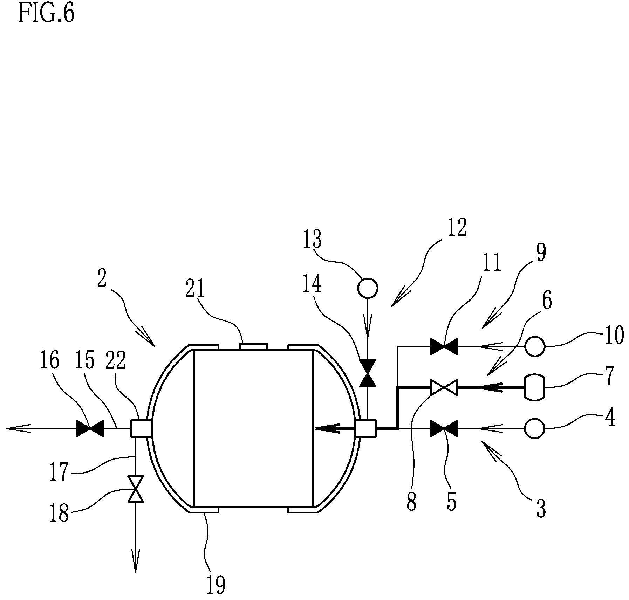

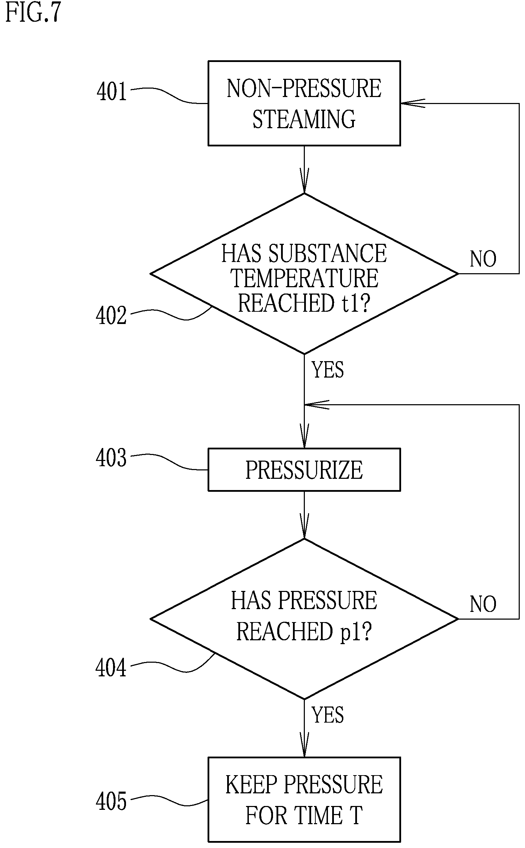

Fig. 6 illustrates the steaming and cooling device 2 in the steaming step (Step 400 in Fig. 2). In the steaming step, steaming is performed while rotating the drum 19 to mix the raw material. The steaming is achieved using steam supplied from a steam supply source 7 of the steam supply device 6 to the interior of the drum 19 via the steam regulating valve 8. In Fig. 6, the supply path of the steam is indicated by a thick line. Fig. 7 is a flowchart specifically illustrating the steaming step. In an early stage of the steaming step, non-pressure steaming is performed with an air release valve 16 opened (Step 401 in Fig. 7), and the air release valve 16 is closed for pressurization when the substance temperature reaches t1 (for example, 100°C) (Steps 402 to 403 in Fig. 7). During pressurization, when the pressure reaches a certain pressure p1 (for example, 0.1 MPa), the pressure is kept for a time T (for example, 40 minutes) (Steps 404 to 405 in Fig. 7).

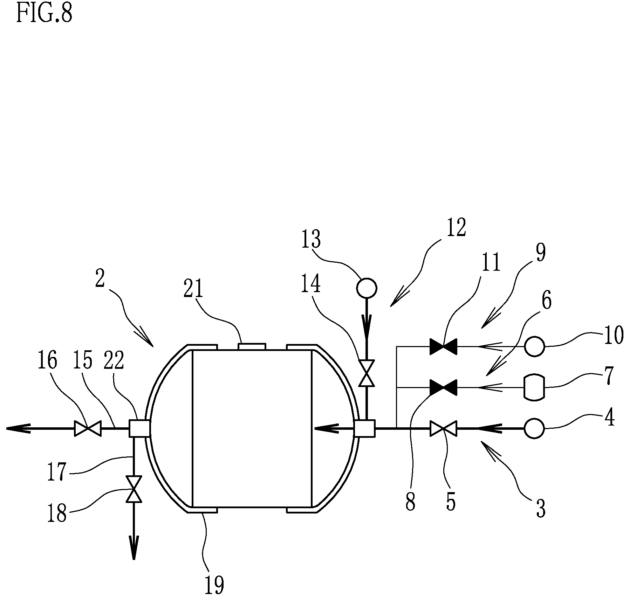

Fig. 8 illustrates the steaming and cooling device 2 in the cooling step (Step 500 in Fig. 2). In the cooling step, cooling is performed while rotating the drum 19 to mix the raw material. First, the air release valve 16 of the drum 19 is opened to release the pressure, and then sterile air is supplied to the interior of the drum 19, and cooling water is supplied to the drum 19. The sterile air is supplied from a sterile air supply source 4 of the raw material processing sterile air supply device 3 to the interior of the drum 19, which is the main body of the steaming and cooling device 2, via the sterile air regulating valve 5. The cooling water is supplied from a cooling water supply source 13 of the cooling water supply device 12 to a jacket (between the inner tank and the outer tank of the drum 19) via the cooling water regulating valve 14. The cooling water is discharged through a water drainage channel 17 with the cooling water regulating valve 18 opened. In Fig. 8, the supply path and discharge path of the sterile air and the supply path and discharge path of the cooling water are indicated by thick lines.

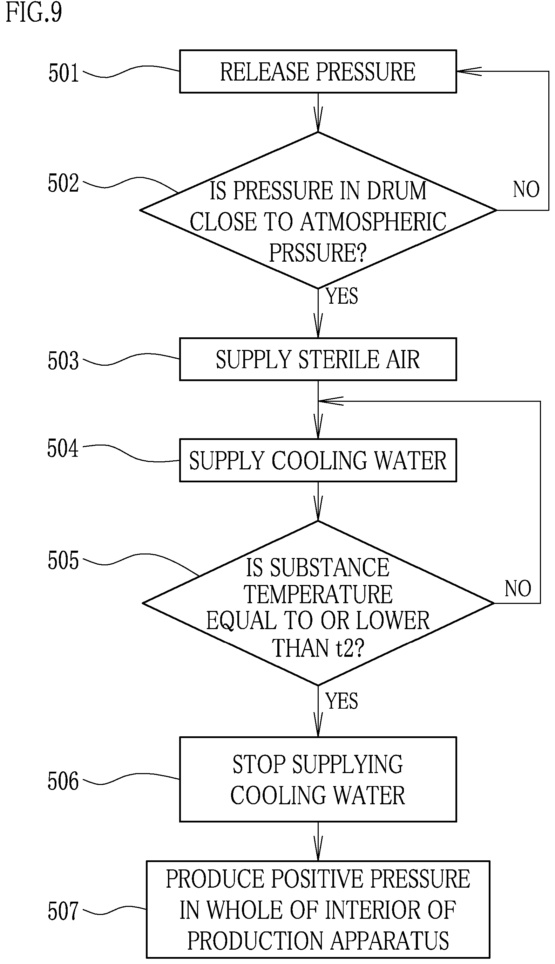

Fig. 9 is a flowchart specifically illustrating the cooling step. In an early stage of the cooling step, the pressure in the drum 19 is released until the pressure is lowered to approximately atmospheric pressure (Steps 501 to 502 in Fig. 9). After that, as described in detail above, sterile air is supplied to the interior of the drum 19 (Step 503 in Fig. 9), and cooling water is supplied to the drum 19 (Step 504 in Fig. 9).

The temperature of the raw material at the time when the pressure releasing ends is about 100°C. After the pressure releasing, in the state in Fig. 8, if the air release valve 16 is fully closed to seal the drum 19, steam from the raw material condenses on the inner surface of the drum 19, and a negative pressure occurs in the drum 19. As a result, the drum may suck outside air in through a tiny clearance in the drum 19, and contamination by Misc. bacteria may occur. Therefore, the interior of the drum 19 is kept at a positive pressure by the raw material processing sterile air supply device 3 supplying sterile air into the drum 19 as described above, thereby preventing a negative pressure from occurring in the drum 19. In this process, the manhole 21 is kept closed.

Before the end of the pressure releasing, in the state in Fig. 8, the degree of opening of the air release valve 16 is preferably set to slightly open so that the interior of the drum 19 is kept at a positive pressure with a minimum supply of sterile air. For example, two air release lines can be provided, one of which is provided with an air release valve with a large diameter, and the other of which is provided with an air release valve with a small diameter. At the start of the pressure releasing, both the two air release valves can be opened to lower the pressure to approximately atmospheric pressure in a short time, and before the end of the pressure releasing, the air release valve with a large diameter can be closed, and the air release valve with a small diameter can be kept open so that the interior of the drum 19 is kept at a positive pressure.

In the cooling step, when the temperature of the cooling water discharged is higher than 40°C, for example, the cooling water is discharged and flow to the floor, while the temperature of the cooling water discharged is equal to or lower than 40°C, the discharge of the cooling water is stopped, and the cooling water is circulated using a chiller.

In an early stage of the cooling step, the temperature of the cooling water discharged is high, so that the cooling water can be collected and used for hot water cleaning of the apparatus, for example. However, when the temperature of the cooling water discharged is lowered and becomes unsuitable for hot water cleaning, the cooling water stops being collected and flowed to a channel for flushing the floor or a channel for cooling water circulation using a chiller, so that the cooling water can be efficiently used.

The supply of the cooling water continues until the substance temperature becomes equal to or lower than t2 (for example, 40°C) (Step 505 in Fig. 9), and when the substance temperature becomes equal to or lower than t2, the supply of the cooling water is stopped (Step 506 in Fig. 9), and a positive pressure is produced in the drum 19 (Step 507 in Fig. 9). In Fig. 1, a sealant is used between the conveyor device 36 and the mixing device 31 coupled to each other, between the mixing device 31 and the inoculating device 40 coupled to each other, and between the mixing device 31 and the ventilated solid cultivation device 50 coupled to each other. The steaming and cooling device 2 and the conveyor device 36 are also coupled to each other with a sealant. With such a configuration, the culture sterile air supply device 70 supplies sterile air into the ventilated solid cultivation device 50, and the raw material processing sterile air supply device 3 supplies sterile air into the drum 19, so that the whole of the interior of the producing apparatus 1 can be kept at a positive pressure and the raw material can be prevented from coming into contact with the outside air.

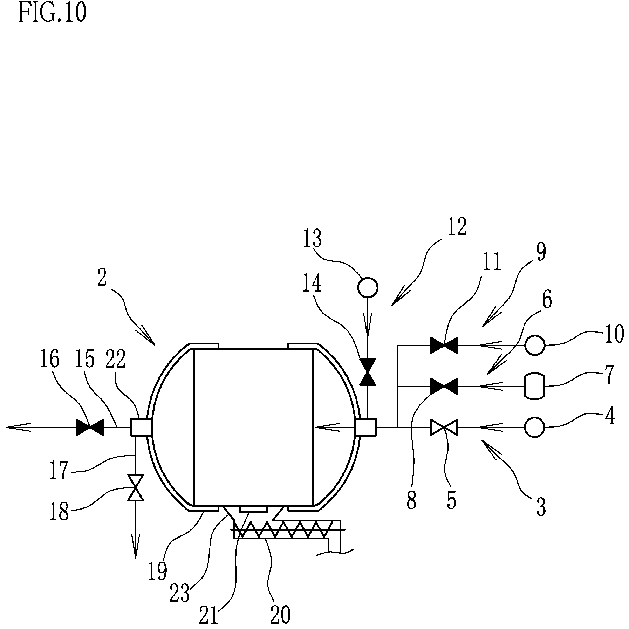

Fig. 10 illustrates the steaming and cooling device 2 when a positive pressure is produced in the whole of the interior of the producing apparatus 1. The lid of the manhole 21 is removed when the manhole 21 is located at the top, and in the state in Fig. 10, the manhole 21 is open. Although the lid is manually removed, the part including the manhole 21 is in a clean room (not shown). When opening the manhole 21, the degree of opening of the air release valve 16 is set to slightly open, and the raw material processing sterile air supply device 3 supplies sterile air into the drum 19 to keep the interior of the drum 19 at a positive pressure, so that contamination by Misc. bacteria does not occur. After the manhole 21 is opened, the air release valve 16 is closed, and the supply of sterile air is continued.

In the state in Fig. 10, the manhole 21 is in a discharge hopper 23, and the drum 19 and the discharge hopper 23 are coupled to each other with a sealant. An exhaust duct 61 of the ventilated solid cultivation device 50 has a flapper 65 that opens in response to a pressure higher than a certain pressure. As described above, a positive pressure can be produced in the whole of the interior of the producing apparatus 1 by the culture sterile air supply device 70 supplying sterile air into the ventilated solid cultivation device 50 illustrated in Fig. 1 and the raw material processing sterile air supply device 3 supplying sterile air into the drum 19.

The process proceeds from the state in Fig. 10 to the heaping step (Step 600 in Fig. 2). The heaping step is a step of conveying and heaping the raw material onto the culture bed 52 in the ventilated solid cultivation device 50. Since the manhole 21 is open in the state in Fig 10, the raw material in the drum 19 is discharged into the discharge hopper 23. The raw material is then supplied to the heaping device 30 illustrated in Fig. 1. More specifically, in Fig. 1, the raw material is conveyed by the conveyor device 36, which is a part of the heaping device 30, and supplied to the mixing device 31, which is also a part of the heaping device 30.

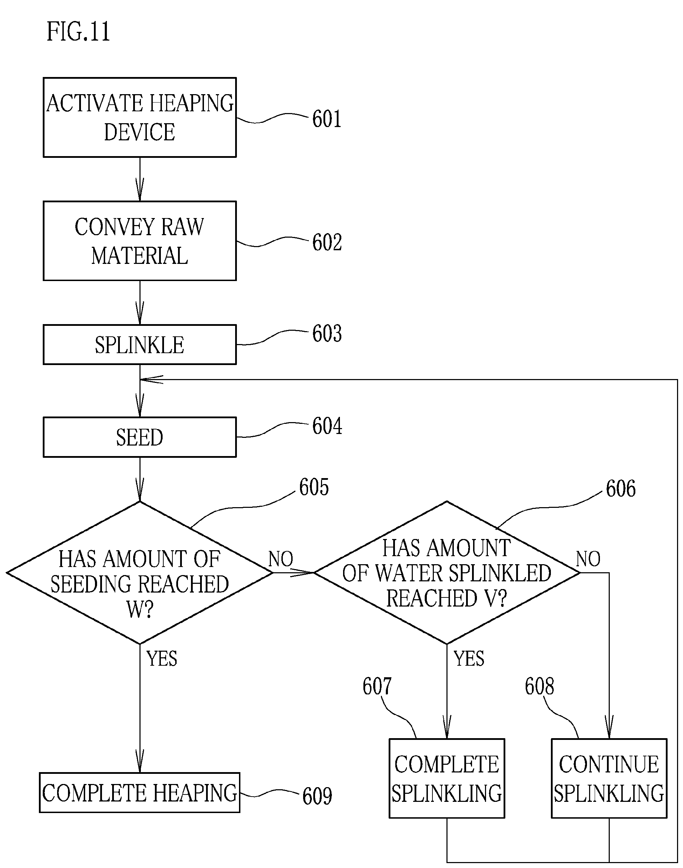

In the following, the heaping step will be described with reference to Figs. 1 and 11. Fig. 11 is a flowchart specifically illustrating the heaping step. In Fig. 1, a mixing screw 35 and a conveyor screw 37 are first rotated (Step 601 in Fig. 11). The drum 19 is then rotated to discharge the raw material into the conveyor device 36 through the manhole 21 so that the raw material is conveyed (Step 602 in Fig. 11). While the raw material is conveyed by the water adding device 32 for the heaping device, water is splinkled onto the raw material until the amount of water splinkled reaches V (for example, 1000 L) (Steps 603 to 608 in Fig. 11). The splinkling is achieved using water supplied from a water supply source 33 via a water supply valve 34.

More specifically, the amount of water splinkled is adjusted so that the water content of the raw material immediately after the heaping is 65% for example. Furthermore, contamination by Misc. bacteria during culture is prevented by using pH-adjusted water as the water to be splinkled so that the pH of the raw material immediately after the heaping is 4.2, for example. In the heaping step, if the water content of the raw material immediately after the heaping is too high, the risk of contamination by Misc. bacteria in the subsequent culture step increases, while if the water content of the raw material is too low, the raw material is not suitable for growth or sporulation of the fungus. Therefore, a target value of the water content of the raw material immediately after the heaping preferably falls within a range from 30% to 75%. When the main ingredient of the raw material is bran, the target value of the water content of the raw material preferably falls within a range from 60% to 70%.

In addition, the seed mold can be produced in a more aseptic condition by appropriately controlling the pH of the raw material. If the pH of the raw material immediately after the heaping is high, the risk of contamination by Misc. bacteria increases, and if the pH is too low, the raw material is not suitable for growth or sporulation of the fungus. Therefore, a target value of the pH of the raw material immediately after the heaping preferably falls within a range from 3.0 to 6.0, and more preferably falls within a range from 3.5 to 5.5.

The pH adjuster is not particularly limited, and lactic acid for brewing, which is inexpensive and non-volatile, can be used, for example. Although the pH adjustment of the raw material can be performed in the drum 19 by the water adding device 9 for the steaming and cooling device in the splinkling and mixing step (Step 300 in Fig. 2), the pH adjustment is preferably performed by the heaping device 30, since the temperature in the drum 19 in the subsequent steaming step (Step 400 in Fig. 2) is high, and any low-pH water remaining in a clearance in the drum 19 may cause corrosion of the apparatus.

Onto the raw material with water splinkled thereon, the original seed mold from a original seed mold container 41 is then conveyed by a conveyor screw 42 in the inoculating device 40, and inoculating is performed until the amount of inoculating reaches W (Steps 604 to 605 in Fig. 11). When the amount of inoculating reaches W, the heaping completes (Step 609 in Fig. 11). Meanwhile, the splinkling is continued until the amount of water splinkled reaches V (for example, 1000 L) (Steps 606 and 608 in Fig. 11), and the splinkling completes when the amount of water splinkled reaches V (Step 607 in Fig. 11).

In this embodiment, the original seed mold container 41 having an airtight structure and an outlet port of the conveyor screw 42 are coupled to each other by an equal pressure line 43, and the original seed mold can be stably supplied even in a condition where the whole of the interior of the producing apparatus 1 is kept at a positive pressure. The original seed mold used in this embodiment is Aspergillus sojae strain. However, any strain suitable as a seed mold, such as Aspergillus oryzae strain, can be used.

In Fig. 1, the water adding device 32 for the heaping device and the inoculating device 40 are arranged so that the inoculating is performed after the splinkling. However, the present disclosure is not limited to this arrangement, and these devices may be arranged in the reverse order. Furthermore, the inoculating may be performed in the drum 19 in advance.

When the heaping step completes, the heaping of the raw material 60 onto the culture bed 52 in the ventilated solid cultivation device 50 completes. The accumulation height of the raw material 60 on the culture bed 52 after the heaping may fall within a range from 100 mm to 500 mm and is 250 mm, for example. After the completion of the heaping step, the process proceeds to a culture step (Step 700 in Fig. 2).

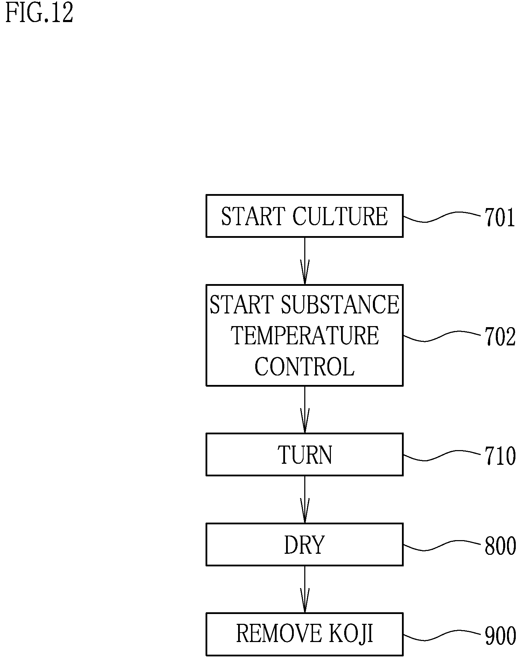

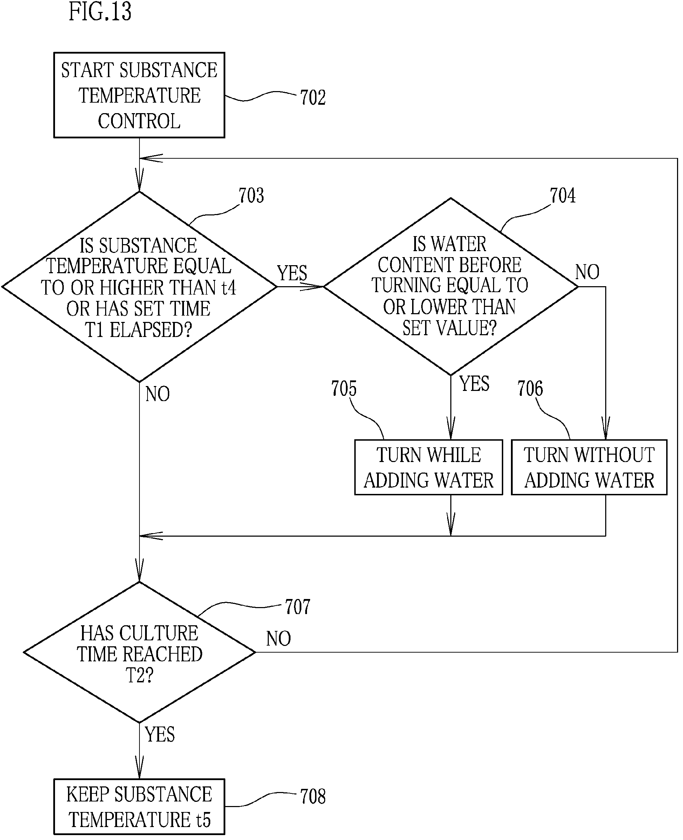

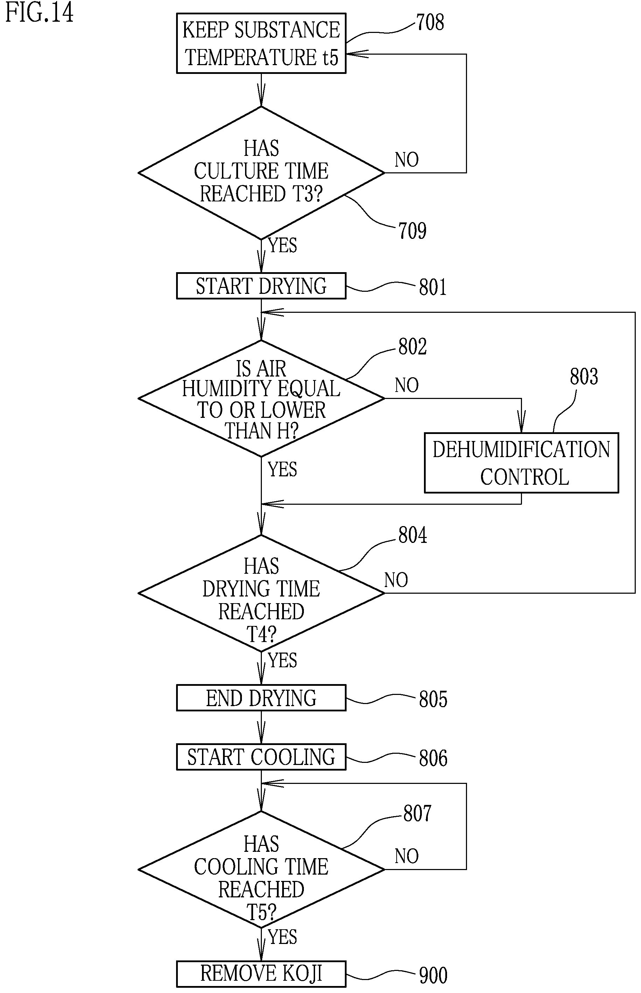

In the following, the culture step and a drying step will be described with reference to Figs. 1, 2 and 12 to 14. As illustrated in Fig. 2, after the end of the culture step (Step 700), the process proceeds to a drying step (Step 800). Fig. 12 is a flowchart illustrating an overview of the culture step and the drying step. Fig. 13 is a flowchart specifically illustrating substance temperature control in the culture step. Fig. 14 is a flowchart specifically illustrating the drying step following the culture step.

In Fig. 12, when culture starts (Step 701 in Fig. 12), substance temperature control also starts (Step 702 in Fig. 12). The substance temperature control is performed by the culture sterile air supply device 70 supplying sterile air into the ventilated solid cultivation device 50 illustrated in Fig. 1. Although the supplying of the sterile air in the culture step primarily aims to control the substance temperature of the raw material 60 being cultured to a target substance temperature, the supplying of the sterile air also prevents entry of Misc. bacteria by producing a positive pressure in a culture device main body 51.

In the culture step, there is no need to produce a positive pressure in the whole of the interior of the production apparatus 1, as far as at least the culture device main body 51 is kept at a positive pressure. For example, the pressure in the drum 19 during cleaning need not be positive. In Fig. 1, a positive pressure can be produced in the culture device main body 51 alone by pulling and retracting the mixing device 31 out of the culture device main body 51 and closing the opening of the culture device main body 51 resulting from the retraction.

In Fig. 1, the culture sterile air supply device 70 includes a sterilization filter 71, an ozone supply source 72, an air conditioner 73 and an air ventilator 74, and can supply sterile air into the ventilated solid cultivation device 50 and control the air temperature and air humidity of the supplied sterile air. In this way, the substance temperature is controlled to be a set value. If the relative humidity of the sterile air is too high, fine water droplets are formed on a lower part of the raw material 60, and the possibility of contamination by Misc. bacteria increases. If the relative humidity of the sterile air is too low, the raw material is not suitable for growth and sporulation of the fungus. Therefore, the relative humidity RH of the sterile air preferably falls within a range from 70% to 99%, and the relative humidity RH more preferably falls within a range from 90% to 98%.

Since the culture sterile air supply device 70 includes the air ventilator 74, the linear velocity of the sterile air passing through the raw material 60 on the culture bed 52 can be controlled. The linear velocity is changed depending on the condition of the culture as required. If the linear velocity is too high, the raw material 60 is ventilated, and spores formed are scattered. If the linear velocity is too low, the substance temperature significantly varies with the depth in the accumulated raw material, and the seed mold cannot be uniformly produced. Therefore, it is preferable to change the linear velocity as required during the culture.

Although not shown in Fig. 1, the culture sterile air supply device 70 is provided with a shut-off valve between the air conditioner 73 and the sterilization filter 71 in order to prevent steam from reaching the sterilization filter 71 to make the sterilization filter 71 unusable during steam sterilization of the ventilated solid cultivation device 50. On this occasion, the part upstream of the shut-off valve is sterilized by ozone from the ozone supply source 72.

In this embodiment, air conditioning of the interior of the ventilated solid cultivation device 50 by supplying sterile air is one-way, and all of the air having passed through the raw material 60 is discharged to the outside of the apparatus through the exhaust duct 61. By adopting the one-way system, the culture can be achieved in a more aseptic condition than the circulation system. As illustrated in Fig. 1, in the exhaust duct 61, water from a water supply source 63 is sprayed from a spray nozzle 62 via a water regulating valve 64 to prevent spores from flying to the outside.

During the substance temperature control, turning of the raw material 60 (Step 710 in Fig. 12) is performed. Referring to Fig. 1, the turning is achieved by lowering the turning machine 58 while rotating the culture bed 52 about the central post 53 and rotating the turning machine 58 to turn the raw material 60. In the culture step, the raw material 60 tends to form lumps due to the growth of the koji mold, and it would be difficult to control the substance temperature to an intended temperature if the raw material 60 is left alone. Therefore, turning is regularly performed to turn the raw material 60.

In addition, the water content of the raw material 60 gradually decreases in the course of the culture step and becomes unsuitable for sporulation. Therefore, in the turning, water is splinkled to control the water content of the raw material. The splinkling is performed by the water adding device 55 for culture illustrated in Fig. 1. Water from a water supply source 56 is splinkled to the raw material 60 via a water regulating valve 57. Furthermore, the pH of the raw material 60 gradually varies in the course of the culture step, and the risk of contamination by Misc. bacteria increases. Therefore, in the culture step, as in the heaping step, the pH of the water splinkled is adjusted to prevent contamination by Misc. bacteria.

In Fig. 13, after the start of the substance temperature control (Step 702 in Fig. 13), it is determined whether or not the substance temperature is equal to or higher than t4 (for example, 36°C) or whether or not the culture time has reached a set time T1 (Step 703 in Fig. 13). When the substance temperature is equal to or higher than t4, turning is performed. Even when the substance temperature is lower than t4, turning is performed if the culture time has reached the set time T1.

Specifically, in Step 704 in Fig. 13, it is determined whether or not the water content of the raw material before turning is equal to or lower than a set value (for example, 65%). If the water content is equal to or lower than the set value, turning is performed by the water adding device 55 for culture adding water, aiming at a water content of 65%, for example (Step 705 in Fig. 13). If the water content is not equal to or lower than the set value, turning is performed without adding water (Step 706 in Fig. 13). For example, six turnings are performed according to different set times T1: a first turning is performed when the culture time has reached 16 hours, a second turning is performed when the culture time has reached 21 hours, a third turning is performed when the culture time has reached 24 hours, a fourth turning is performed when the culture time has reached 30 hours, a fifth turning is performed when the culture time has reached 38 hours, and a sixth turning is performed when the culture time has reached 46 hours. Set time T2, which is 48 hours here, and no turning is performed after that.

After that, until the culture time reaches the set time T2, the substance temperature control is performed by repeating turnings while determining whether the culture time has reached set times T1 (T1-1, T1-2, T1-3, T1-4, T1-5, and T1-6) (Step 703 in Fig. 13). After the culture time reaches T2, the substance temperature is controlled to be kept at an substance temperature t5 (for example, 30°C) (Step 708 in Fig. 13).

In the following, the drying step will be described with reference to Fig. 14. Drying can prevent growth of Misc. bacteria and allow long-term storage. For the sake of convenience, Fig. 14 also illustrates Step 708 in Fig. 13. As described above, in Fig. 13, once the culture time reaches the set time T2 (for example, 48 hours), the substance temperature t5 (for example, 30°C) is maintained. Then, as illustrated in Fig. 14, when the culture time reaches a set time T3 (for example, 72 hours) from the start of culturing, drying is started (Steps 709 to 801 in Fig. 14).

After the start of the drying, by setting the air temperature at 40°C, for example, dehumidification control is performed until the air humidity becomes equal to or lower than H (for example, RH becomes equal to or lower than 35%) (Steps 802 to 803 in Fig. 14). When the drying time reaches T4 (for example, 20 hours), the drying is ended (Steps 804 to 805 in Fig. 14). After the end of the drying, cooling is started by setting the air temperature at 20°C, for example (Step 806 in Fig. 14). In Step 807 in Fig. 14, it is determined whether or not the cooling time has reached T5 (for example, 1 hour), and when the cooling time has reached T5, the cooling is ended, and koji removal is performed to remove the produced seed mold from the ventilated solid cultivation device 50 (Step 900 in Fig. 14). Referring to Fig. 1, the koji removal is achieved by lowering the discharging screw 54 while rotating the culture bed 52 about the central post 53 and conveying the produced seed mold toward a discharge chute 59 by rotation of a screw.

As described above, the ventilated solid cultivation device 50 includes the turning machine 58 and the discharging screw 54. These devices are preferably of the swing type, rather than the lift type. The lift type is not suitable for steam sterilization, since steam leakage occurs at a sliding part for lifting and lowering. The swing type allows steam sterilization, so that the culture can be performed in a more aseptic condition.

An embodiment of the present disclosure has been described above. The present disclosure provides various effects as described below, and allows efficient production of seed mold. The production apparatus 1 according to the present disclosure generally includes the steaming and cooling device 2, the heaping device 30 and the ventilated solid cultivation device 50. The batch steaming and cooling of the raw material by the steaming and cooling device 2 can be automated, and the heaping of the raw material by the heaping device 30 can also be automated. In the ventilated solid cultivation device 50, the ventilation of the raw material 60 on the culture bed can be automated, and the koji removal can also be automated with the discharging screw 54. That is, each device forming the production apparatus 1 can be automated, and in particular, the heaping and the koji removal can be automated. The production apparatus 1 formed by coupling these devices is suitable for automation.

The culture of the raw material 60 is performed by using the ventilated solid cultivation device 50 and accumulating the raw material 60 on the culture bed 52. Therefore, the accumulation height of the raw material 60 can be increased, and the present disclosure is suitable for mass production of seed mold. In addition, since sterile air passes through the raw material 60 on the culture bed 52 during the culture, the temperature can be controlled to reduce the variation of the substance temperature with the depth in the accumulation, by controlling the air temperature and air humidity of the sterile air. In addition, since the raw material 60 on the culture bed 52 can be turned, the variation of the number of spores in the produced seed mold in the depth direction of the accumulation can be reduced.

Since the production apparatus 1 according to the present disclosure includes the water adding device 55 for culture that supplies water to the raw material 60 on the culture bed 52, the water content of the raw material can be appropriately adjusted, and a seed mold containing a larger number of spores can be produced. In addition, since the heaping device 30 and the ventilated solid cultivation device 50 are separate devices, the raw material 60 can be uniformly seeded in the heaping device 30 in advance and then be heaped to the ventilated solid cultivation device 50.

In the following, the culture step will be described in more detail with reference to examples. In an example 1, 2000 kg of bran was subjected to splinkling processing aiming at a water content of the raw material of 55%, steaming under pressure, cooling, inoculating with Aspergillus sojae strain for soy source as a original seed mold, splinkling aiming at a water content of the raw material of 65% and a pH of the raw material of 4.2 immediately after heaping, and then 72 hours of culture. The accumulation height immediately after heaping was 250 mm. In the example 1, depending on the water content of the raw material before turning, the water content was adjusted during turning. Table 1 below illustrates a relationship between the elapsed time and the water content and the pH of the raw material.

**[Table 1]**

| Elapsed time | Turning | Water content before turning | pH before splinkling | pH after splinkling |
|---|---|---|---|---|
| 0 Hours | (Start of culture) | 65.2% | 4.2(No splinkling) | |
| 16 Hours | First turning | 64.3% | 4.6(No splinkling) | |
| 21 Hours | Second turning | 62.5% | 4.9 | 4.7 |
| 24 Hours | Third turning | 63.0% | 5.3 | 5.0 |
| 30 Hours | Fourth turning | 57.0% | 5.9 | 4.9 |
| 38 Hours | Fifth turning | 55.4% | 6.2 | 4.8 |
| 46 Hours | Sixth turning | 57.6% | 6.8 | 5.7 |
| 72 Hours | (End of culture) | 56.0% | 7.0(No splinkling) | |

In Table 1, at the start of the culture, the water content of the raw material was 65.2%, and the pH was 4.2. After 16 hours from the start of the culture, the first turning was performed. The water content was as high as 64.3%, and splinkling was not performed. The pH was 4.6 and was slightly higher than that at the start of the culture. After 21 hours from the start of the culture, the second turning was performed. The water content was lowered to 62.5%, and splinkling of water whose pH was adjusted to 2.0 was performed aiming at a water content of 65%. The pH of the raw material before the splinkling was 4.9, and the pH was slightly lowered to 4.7 as a result of the splinkling. In the subsequent third to sixth turnings, splinkling of water whose pH was adjusted to 2.0 was performed aiming at a water content of 65%.

At 46 hours after the start of the culture, sporulation was observed to some extent. After the sixth turning, no turning was performed, since the koji would no longer generate much heat, and in order to prevent formed spores from being ventilated.

At 72 hours after the start of the culture, the culture was ended. The seed mold at the end of the culture was green or light green and soft, and sufficient sporulation was observed. In addition, there was no vertical unevenness of sporulation in the height direction of the accumulation.

The result of measurement of the number of spores was as follows: in the height direction of the accumulation, the number of spores was 11.2 billion spores/gram of dry seed mold in an upper part, 10.9 billion spores/gram of dry seed mold in a middle part, and 11.1 billion spores/gram of dry seed mold in a lower part. As illustrated in Table 1, at the end of the culture, the water content of the whole of the seed mold was 56.0%, and the pH was 7.0. The number of spores was measured in a common measurement method using a hemacytometer. Although the water content of the seed mold at the end of the culture was as high as 56.0% in this example, the water content varies with the culture condition or the amount of water splinkled during the culture, and may be 50%. Therefore, in order to exclude the influence of the water content for proper evaluation, the number of spores and the number of Misc. bacteria were converted to a value per gram of dry seed mold.

In the example 1, in the drying step, the air temperature was 40°C, the relative humidity RH was 35%, and the linear velocity of the sterile air passing through the raw material was 0.09 m/s. After 20 hours from the start of the drying, the water content of the whole of the raw material was 7.2%.

In the following, effects of the present disclosure will be described by referring to test results. Table 2 below illustrates the number of Misc. bacteria in the finished koji and the number of spores in the finished koji in the example 1 and a comparative example 1. In Table 2, "not detected" means 1 × 10¹ (number of Misc. bacteria/gram of dry seed mold) or less (the same holds true for Table 4). The example 1 is ventilated culture using the production apparatus 1 illustrated in Fig. 1, and the comparative example 1 is static culture of a horizontal thin layer. The amount of bran processed is 2000 kg in the example 1 as described above, and is also 2000 kg in the comparative example 1, in which twenty multi-stage static culture apparatuses for horizontal thin layers each of which can handle 100 kg of bran (the apparatus according to Patent Literature 1) were used, and 2000 kg of bran were able to be processed by operating 20 apparatuses.

The conditions were the same in the example 1 and the comparative example 1, except for the difference in scale and the differences between the ventilated culture and the static culture. In both the examples, splinkling processing was performed aiming at a water content of the raw material of 55%, steaming under pressure was performed in a normal manner, cooling and inoculating were then performed, and culture was performed for 72 hours. In the comparative example 1, the heaping of the raw material added with water into the twenty apparatuses and the koji removal were manually performed, and therefore, there was a problem of workability. In the example 1, however, the heaping and the koji removal were automated, and the seed mold was able to be efficiently produced. As illustrated in Table 2, in the example 1, compared with the comparative example 1 of the static culture, a seed mold of similar quality was able to be produced in terms of number of Misc. bacteria and number of spores.

**[Table 2]**

| | Number of spores (billions/g of dry seed mold) | Number of Misc. bacteria (/g of dry seed mold) |
|---|---|---|
| Example 1 (ventilated culture) | 110 | Not detected |
| Comparative example 1 (static culture) | 100 | Not detected |

In the following, effects of the adjustment of the water content of the raw material in the culture step will be described. As described above, the ventilated solid cultivation device 50 includes the water adding device 55 for culture, and can adjust the water content of the raw material. Table 3 below illustrates test results in an example 2 in which the production apparatus 1 was used to appropriately adjust the water content of the raw material and an example 3 in which the same production apparatus 1 was used but the water content was controlled to be low.

**[Table 3]**

| | | Water content by time after start of culture (%) | | | | Number of spores (billions/g of dry seed mold) |
|---|---|---|---|---|---|---|
| | | 0 Hours | 24 Hours | 46Hours | 72 Hours | |
| Example 2 (water content was appropriately adjusted) | Before splinkling | 65.5 | 63.4 | 57.8 | 56.6 | 113 |
| | After splinkling | - | 66.7 | 65.1 | - | |
| Example 3 (water content was controlled to be low) | Before splinkling | 59.9 | 57.7 | 56.2 | 42.7 | 64 |
| | After splinkling | - | 60.5 | 60.7 | - | |

In Table 3, in the example 2, the water content of the raw material was appropriately adjusted so that the water content after the splinkling was about 65%. However, in the example 3, the water content was intentionally controlled to be low so that the water content after the splinkling was about 60%. In both the examples 2 and 3, the pH of the raw material immediately after the heaping was 4.2, pH-adjusted water whose pH was adjusted to keep the pH of the raw material being cultured constant was splinkled in the culture step, and the pH of the raw material immediately after the culture was 7.0. As illustrated in Table 3, in the example 2 in which the water content was appropriately adjusted, the number of spores was obviously higher than that in the example 3 in which the water content was controlled to be low. This means that the production method for a seed mold according to the present disclosure can produce a seed mold containing a larger number of spores by appropriately adjusting the water content of the raw material.

Next, effects of the pH adjustment will be described. Table 4 below illustrates an example 4 in which the production apparatus 1 was used, and the pH of the raw material was adjusted in the heaping step aiming at a pH of 4.2 immediately after the heaping and an example 5 in which the same production apparatus 1 was used, but the amount of lactic acid added was reduced, and the pH adjustment was performed to lower extent. In the culture step, pH-adjusted water whose pH was adjusted to 2.0 was splinkled in the example 4, while water whose pH was not adjusted was splinkled in the example 5. As illustrated in Table 4, in the example 4 in which the pH was appropriately adjusted, the number of Misc. bacteria was less and the number of spores was slightly more than those in the example 5.

**[Table 4]**

| | | pH by time after start of culture | | | | Number of Misc. bacteria (/g of dry seed mold) | Number of spores (billions/g of dry seed mold) |
|---|---|---|---|---|---|---|---|
| | | 0 Hours | 24 Hours | 46 Hours | 72 Hours | | |
| Example 4 | Before splinkling | 4.2 | 4.9 | 6.6 | 7.3 | Not detected | 106 |
| | After splinkling | - | 4.8 | 5.4 | - | | |
| Example 5 | Before splinkling | 5.0 | 5.3 | 7.1 | 7.1 | 2.4×10⁵ | 98 |
| | After splinkling | - | 5.4 | 7.2 | - | | |

In the following, examples 6 to 8 in which the production apparatus 1 illustrated in Fig. 1 was used, and different kinds of original seed molds were used will be described. In the examples 6 to 8, splinkling processing was performed on 2000 kg of bran aiming at a water content of the raw material of 55%, steaming under pressure was performed in a normal manner, cooling and inoculating were then performed, and culture was performed for 72 hours. A original seed mold A in the example 6 was Aspergillus sojae strain for soy sauce, a original seed mold B in the example 7 was Aspergillus sojae strain for miso, and a original seed mold C in the example 8 was Aspergillus oryzae strain for sake. In each example, the water content, the number of spores and the average spore diameter immediately after the culture were measured. Table 5 below illustrates test results in the examples 6 to 8. The spore diameter does not vary with the culture method but is specific to the kind of the original seed mold. Observation of the produced seed mold shows that all of the three kinds of fungi in the examples 6 to 8 substantially assimilated the nutrients of the bran, which was the raw material, and only bran skin was left. The number of spores per gram of dry seed mold significantly varies with the kind of the original seed mold, because the weight of one spore varies with the kind of the original seed mold. That is, regardless of the kind of the original seed mold, the embodiment of the present disclosure allows efficient assimilation of the raw material and increases the number of spores as far as possible in principle.

**[Table 5]**

| | Kind of the original seed mold | Water content immediately after culture(%) | Number of spores (billions/g of seed mold) | Average spore diameter (µm) |
|---|---|---|---|---|
| Example 6 | A | 57.0 | 106 | 4.3 |
| | Sojae for soy sauce | | | |
| Example 7 | B | 55.7 | 93 | 4.6 |
| | Sojae for miso | | | |
| Example 8 | C | 58.4 | 39 | 5.1 |
| | Oryzae for sake | | | |

An embodiment and examples of the present disclosure have been described above. According to the method in the embodiment described above, the steaming and cooling step of steaming and cooling the raw material in batches using the batch steaming and cooling device 2 is performed, and the culture step of culturing the raw material accumulated on the culture bed 52 using the ventilated solid cultivation device 50 that cultures the raw material accumulated on the culture bed 52 capable of being ventilated is performed. Although the ventilated solid cultivation device 50 including the culture bed 52 having a circular shape is used according to the method in the embodiment of the present disclosure described above, a method using a casten-type culture device having a rectangular casten (culture container) in which raw material is placed on a ventilable culture bed may be used.

A seed mold can be produced in a method in which the process from the steaming and cooling step to the culture step is performed in one drum device having a drum that rotates about a horizontal axis. However, compared with the method according to the present disclosure, it is difficult to achieve uniform inoculating and splinkling and to appropriately control the substance temperature.

Although the embodiment has been described with reference to an example in which both the water adding device 9 for the steaming and cooling device and the water adding device 32 for the heaping device are provided, what is required is that splinkling of a set amount of water is completed when the heaping step is completed. Only any one of the water adding device for the steaming and cooling device and the water adding device for the heaping device may splinkle a set amount of water. In that case, the other of the water adding device for the steaming and cooling device and the water adding device for the heaping device can be omitted. Furthermore, a raw material added with water may be supplied to the steaming and cooling device 2. In that case, splinkling by the water adding device for the steaming and cooling device may be omitted.

Furthermore, although the present disclosure relates to a production apparatus for a seed mold and a production method for a seed mold, the kind of the seed mold is not particularly limited, and the effects of the present disclosure described above can be achieved regardless of the kind of the seed mold. The seed mold may be a seed mold for soy sauce, a seed mold for miso, or seed mold for sake, for example (see the examples 6 to 8).

### Reference Signs List

- 1: production apparatus for seed mold
- 2: steaming and cooling device
- 3: raw material processing sterile air supply device
- 4: sterile air supply source
- 5: sterile air regulating valve
- 6: steam supply device
- 7: steam supply source
- 9: water adding device for steaming and cooling device
- 10: water supply source
- 12: cooling water supply device
- 13: cooling water supply source
- 15: air release channel
- 17: cooling water drainage channel
- 30: heaping device
- 31: mixing device
- 32: water adding device for heaping device
- 33: water supply source
- 34: water supply valve
- 36: conveyor device
- 40: inoculating device
- 50: ventilated solid cultivation device
- 52: culture bed
- 53: central post
- 54: discharging screw
- 55: water adding device for culture
- 58: turning machine
- 59: discharge chute
- 60: raw material
- 61: exhaust duct
- 70: culture sterile air supply device
- 73: air conditioner

## Claims

1. A production apparatus for a seed mold, comprising:
a steaming and cooling device that steams and cools a raw material in batches;
a ventilated solid cultivation device that cultures the raw material by accumulating the raw material on a culture bed capable of being ventilated;
a heaping device that conveys the raw material discharged from the steaming and cooling device and supplies the raw material to the ventilated solid cultivation device;
a water adding device for culture that supplies water to the raw material on the culture bed; and
a culture sterile air supply device that ventilates sterile air to the raw material on the culture bed,
wherein the sterile air passes through the raw material on the culture bed.

2. The production apparatus for a seed mold according to claim 1, wherein the steaming and cooling device is of a jacket type including an inner tank and an outer tank surrounding the inner tank, the inner tank being cooled by a coolant supplied between the inner tank and the outer tank.

3. The production apparatus for a seed mold according to claim 1, further comprising:
a raw material processing sterile air supply device that supplies sterile air to the steaming and cooling device.

4. The production apparatus for a seed mold according to claim 1, wherein an interior of the ventilated solid cultivation device is kept at a positive pressure by the sterile air supplied by the culture sterile air supply device.

5. The production apparatus for a seed mold according to claim 3, wherein an interior of the steaming and cooling device is kept at a positive pressure by the sterile air supplied by the raw material processing sterile air supply device.

6. The production apparatus for a seed mold according to claim 1, wherein the seed mold after culture is dried by the sterile air supplied by the culture sterile air supply device.

7. The production apparatus for a seed mold according to claim 1, further comprising:
a turning machine that turns the raw material on the culture bed,
wherein the water adding device for culture supplies water to the raw material while the raw material is turned by the turning machine.

8. The production apparatus for a seed mold according to claim 1, wherein air conditioning of an interior of the ventilated solid cultivation device by supplying the sterile air is one-way.

9. The production apparatus for a seed mold according to claim 1, further comprising:
a water adding device for the steaming and cooling device that splinkles water onto the raw material in the steaming and cooling device or a water adding device for the heaping device that splinkles water onto the raw material in the heaping device,
wherein a water content of the raw material in the steaming and cooling device or the raw material in the heaping device and a water content of the raw material on the culture bed are adjustable by adjusting an amount of water from the water adding device for the steaming and cooling device or the water adding device for the heaping device and an amount of water from the water adding device for culture .

10. The production apparatus for a seed mold according to claim 9, wherein the water adding device for the steaming and cooling device, the water adding device for the heaping and the water adding device for culture are capable of adjusting a pH of water to be splinkled.

11. A production method for a seed mold, comprising:
a steaming and cooling step of steaming and cooling a raw material in batches using a steaming and cooling device;
a culture step of accumulating and culturing the raw material on a culture bed capable of being ventilated using a ventilated solid cultivation device; and
a heaping step of conveying the raw material subjected to the steaming and cooling step and supplying the raw material to the ventilated solid cultivation device using a heaping device, the heaping step being between the steaming and cooling step and the culture step;
wherein water is supplied to the raw material on the culture bed in the culture step, and
sterile air is passed through the raw material on the culture bed.

12. The production method for a seed mold according to claim 11, wherein the steaming and cooling device used in the steaming and cooling step is of a jacket type including an inner tank and an outer tank surrounding the inner tank, the inner tank being cooled by a coolant supplied between the inner tank and the outer tank.

13. The production method for a seed mold according to claim 11, wherein sterile air is supplied to the raw material in the steaming and cooling step.

14. The production method for a seed mold according to claim 11, wherein an interior of the ventilated solid cultivation device is kept at a positive pressure by supplying the sterile air to the interior of the ventilated solid cultivation device.

15. The production method for a seed mold according to claim 13, wherein an interior of the steaming and cooling device is kept at a positive pressure by supplying the sterile air to the interior of the steaming and cooling device.

16. The production method for a seed mold according to claim 11, wherein the seed mold after culture is dried by supplying the sterile air.

17. The production method for a seed mold according to claim 11, wherein water is supplied to the raw material while turning is performed to turn the raw material on the culture bed.

18. The production method for a seed mold according to claim 11, wherein air conditioning of an interior of the ventilated solid cultivation device by supplying the sterile air is one-way.

19. The production method for a seed mold according to claim 11, further comprising:
a step of supplying water to the raw material in the steaming and cooling step or a step of supplying water to the raw material in the heaping step,
wherein a water content of the raw material in the steaming and cooling step or the raw material in the heaping step and a water content of the raw material in the culture step are adjustable by adjusting an amount of the water supplied in the steaming and cooling step or an amount of the water supplied in the heaping step and an amount of the water supplied in the culture step.

20. The production method for a seed mold according to claim 19, wherein a pH of the water supplied in the steaming and cooling step, the heaping step and the culture step is adjusted.
